# EUROPEAN PATENT SPECIFICATION

(11) **EP 4 355 397 B1**
(45) Date of publication and mention of the grant of the patent: **25.06.2025**
(21) Application number: 22726675.6
(22) Date of filing: 29.04.2022
(51) Int. Cl.: A61M 16/00, A61M 11/00

(54) **A VENTILATOR SYSTEM**
VENTILATORSYSTEM
SYSTÈME DE VENTILATEUR

(30) Priority: 16.06.2021 EP 21179828
(43) Date of publication of application: 24.04.2024
(73) Proprietor: Stamford Devices Limited, H91 HE94 Galway (IE)
(72) Inventor: GREHAN, Joseph, Galway Business Park, Dangan Galway, H91 HE94 (IE); CASEY, Micheal, Galway Business Park, Dangan, Galway, H91 HE94 (IE); CONNOLLY, Diarmuid, Galway Business Park, Dangan, Galway, H91 HE94 (IE)
(74) Representative: Weldon O'Brien Ltd.
(86) International application number: PCT/EP2022/061596
(87) International publication number: WO 2022/263049

(56) References cited:
- EP-A1- 2 844 321
- WO-A1-2009/118717
- WO-A1-2017/192767
- US-A1- 2005 217 666
- US-B2- 8 336 545
- US-B2- 8 939 152

## Description

### Introduction

The present invention relates to ventilator systems with aerosol treatment.

A nebulizer head is described in our previous PCT application WO2012/046220.

US2005/0217666A describes a method of treating a patient with a pulmonary disease, including delivering a dose of aerosolized medicament intermittently to a ventilator circuit coupled to the respiratory system of the patient. In an operation mode, a controller coupled to an aerosol generator is operable in that an on/off operation of the aerosol is triggered by the controller receiving an external signal, such as a signal from the ventilator, which can correspond to the point in the ventilator's cycle of that is the start of an inhalation phase in which the ventilator begins to push inspiratory air into the ventilator circuit.

WO2017/192767A1 describes a droplet delivery device and related methods for delivering dosages to a subject for pulmonary use.

US8336545B2 describes a method of treating a patient with a pulmonary disease, where the method includes delivering a dose of aerosolized medicament intermittently to a ventilator circuit coupled to the respiratory system of the patient.

An object of the invention is to achieve enhanced versatility in control of nebulizer operation for treatment including both ventilation and aerosol therapy.

### Summary of the Invention

The invention is defined in the appended independent claim 1. Preferred embodiments are matter of the dependent claims.

The invention provides a ventilator system as set out in claims 1 to 9, a method of operating a ventilator system as set out in claims 10 to 14, and a non-transitory storage medium as set out in claim 15.

### Detailed Description of the Invention

The invention will be more clearly understood from the following description of some embodiments thereof, given by way of example only with reference to the accompanying drawings in which:
Fig. 1 is a diagram illustrating a ventilator system of the invention;
Fig. 2 shows an integration interface between a ventilator and a nebulizer of the system;
Fig. 3 is a block diagram illustrating the interface in more detail at a functional level; and
Fig. 4 is a state diagram for operation of the system with signals communicated via the interface.

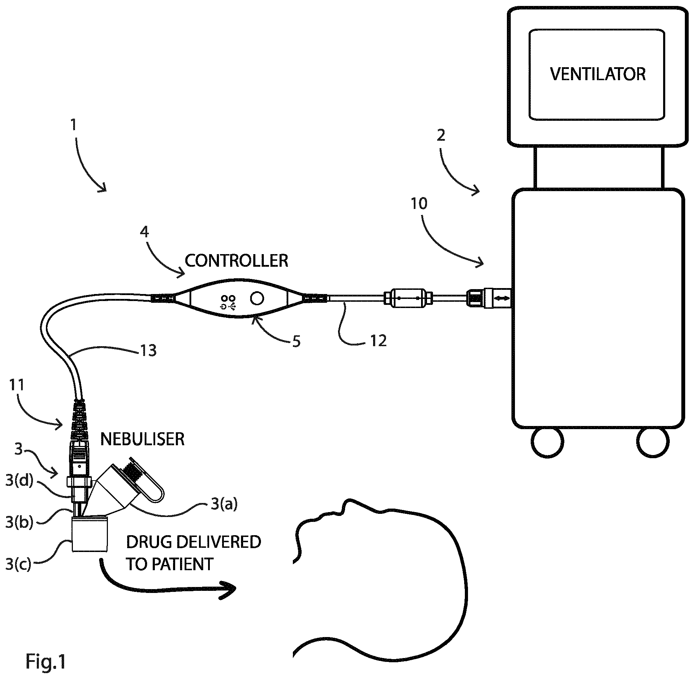

Referring to Fig. 1 a ventilator system 1 comprises a ventilator 2 and a nebulizer 3 which at the physical level is of the type marketed by Aerogen^{™} under the trademark Solo^{™}. Both the ventilator and the nebulizer incorporate digital controllers as is known in the art. An example of the nebulizer head is described in our previous PCT application WO2012/046220. The nebulizer 3 comprises a vibratable aperture plate, and a piezoelectric vibration generator mounted to a support washer. A housing 3(a) delivers liquid medication onto an aerosol generator 3(b) with an aperture plate, which is vibrated at a frequency in the region of 128kHz so that the oscillations provide aerosol droplets having a size in the range of 1µm to 10 µm, for example in the region of 6µm. The droplets exit through an aerosol delivery conduit 3(c). The delivery is through a face mask. The patient interfaces of the ventilator 2 are conventional and are not shown.

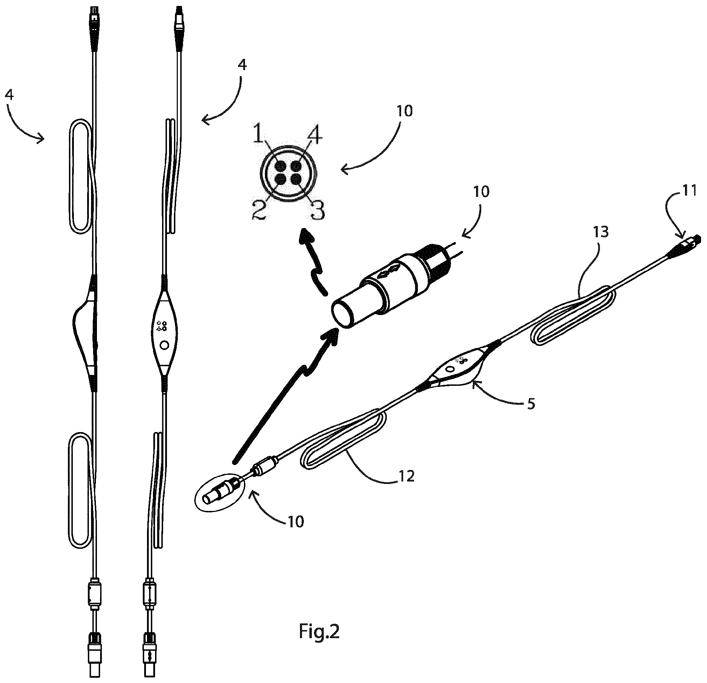

The system 1 also comprises an interface 4 between the ventilator 2 and the nebulizer 3, shown in more detail in Fig. 2. The interface 4 is wired, with the following.
An interface controller 5, having digital data processors, which controls the nebulizer 3 in response to signals which are received from the ventilator 2 controller, and using its stored instructions for optimum nebulization. The interface controller 5 has a housing with LED status lamps.
A ventilator connector 10, which as shown in Fig. 2, is 4-core.
A nebulizer connector 11.
A cable 12 between the ventilator coupler 10 and the interface controller 5, and a cable 13 between the interface controller 5 and the nebulizer coupler 11.

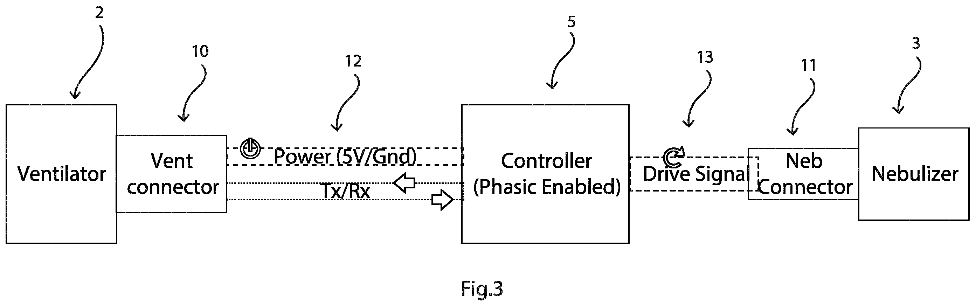

Referring to Fig. 3 the functional components of the interface 4 are shown in more detail, and like parts are given the same reference numerals. The cable 13 transmits nebulizer drive signals, and the cable 12 transmits control signals bi-directionally and also transmits power from the ventilator 2 controller. The system 1 operates with the ventilator 2 and the nebulizer 3 operating in synchronism for optimum patient treatment.

The interface controller 5 comprises a boost circuit with a high frequency, high efficiency DC-to-DC converter with an integrated power switch capable of providing an output voltage and current profile suitable to drive the nebulizer load. There is also a drive circuit utilizing a series inductor to generate alternating AC voltage. The drive circuit includes a high speed MOSFET driver controlled by a pulse width modulated signal from the microcontroller. There is also a microcontroller with an integrated peripheral module that can automatically change clock sources and power levels according to signals received from the ventilator controller. The latter provides power and control signals via the cable 12. The interface controller 5 interprets signals from the ventilator to provide signals on the cable 13 for control of the nebulizer aperture plate. The controller 5 and the nebulizer 3 require no more than 500 mA at nominal 5V to generate a desired aerosol.

The nebulizer drive circuit has components to generate an output sine waveform of approximately 100V AC, causing aerosol to be generated. It uses inputs from the interface controller 5 microcontroller and the boost circuit 10 to achieve its output. The interface controller 5 drive circuit is matched to the impedance of a piezoelectric vibration generator.

The microcontroller of the interface controller 5 generates a square waveform of 120 to 150 kHz which is sent to the drive circuit coupler 11, and it has a boost circuit which generates a nominal 9V to 12V, nominal 9.5V, voltage required by the interface controller 5 from an input within the range of 4.75V to 5.25 V DC. A drive frequency of 120 kHz to 150 kHz is generated to drive the aperture plate at close to its resonant frequency so that enough amplitude is generated to break off droplets and produce the aerosol. The aerosol generator 3(b) may be calibrated at a certain pulse rate by measuring how long it takes to deliver a known quantity of liquid medication. There is a linear relationship between the pulse rate and the nebulizer flow rate, allowing accurate control over the delivery rate. The interface controller 5, using signals from the ventilator 2, can achieve very wide-ranging control of the nebulizer 3.

The interface controller 5 can upload in various embodiments some or all of the following nebulizer characteristics to the host ventilator controller: power consumption, wet/dry state, nebulizer disconnect status, cable disconnect status, error or fault states, nebulization duration, and time of nebulization. The ventilator controller can in various embodiments provide one or more of the following instructions to the interface controller 5: nebulization start/stop, nebulization time, nebulization flow rate, nebulization pulse rate, inspiratory/expiratory signal to enable phased nebulization. The interface controller 5 may operate as a slave device, with a dosing regime determined by the ventilator controller. This allows comprehensive control and treatment monitoring for a wide variety of situations such as in the home or in hospitals. Because the controller is essentially part of the cabling it may not be by-passed accidentally.

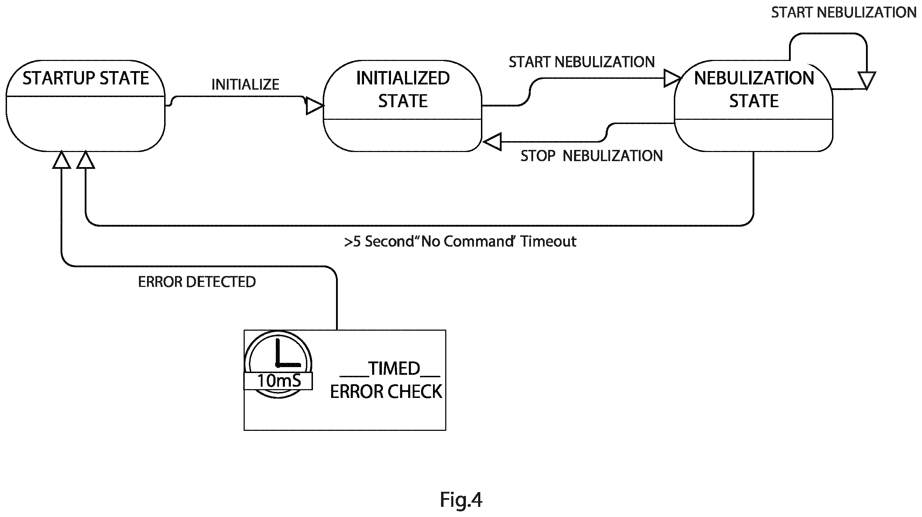

Referring to Fig. 4 a state diagram illustrates the states for operation of the interface controller 5:
Start-up State,
Initialized State, and
Nebulization State.

The Nebulization State ends if there is no command from the coupler 10 for a timeout period of, in one example, 5s. The time-out period of 5 seconds is implemented in software and is restarted each time a valid command from the ventilator 2 is received. Delivery is suspended if a valid Start command is not received within the timeout period and the controller will revert to the initialized state. This feature prevents in delivery of medication, in the case where the ventilator fails to communicate with the controller

Advantageously, the nebulizer 3 is external to the ventilator 2 but yet can be controlled by the ventilator. The interface 4 has the ability to communicate and exchange information with the ventilator 2 to allow phasic delivery, and the display of nebulizer operation information on the ventilator. The interface controller 5 operates external to the ventilator 2, while allowing full control by the ventilator GUI (graphical user interface), solving the issue of restricted space within the ventilator.

The interface 4 provides multiple communication methods to allow flexibility of operation from multiple ventilator/manufacturers. For example, the aerosol delivery device can function with three different types of communication methods, such as full RS232, vendor-specific UART, or Logic level Enable/fault, using a standard command protocol.

Phasic operation by a ventilator of the nebulizer is communicated to the nebulizer 3. Also, the interface controller 5 performs recognition of approved nebulizer types and prevention of use of third party non-approved nebulizers

The interface 4 allows a generic communication protocol to be developed, allowing more versatile use of nebulizers with different types of ventilator. In addition to continuation of drug delivery, it is also crucial that the operational commands (On/Off/Status requests) remain functioning.

The interface 4 detects the type of nebulizer attached and only operates with allowed nebulizer types according to its configuration.

The invention is not limited to the embodiments described but may be varied in construction and detail within the scope of the claims. It is envisaged that the controller may chop the frequency such that aerosol is generated for a short time and then stopped for a short time this gives good control of the nebulizer's flow rate. This lower frequency is called the "pulse rate". The drive frequency may be started and stopped as required using the microcontroller interface controller 5, allowing for control of flow rate by driving the aerosol generator 3(b) for any required pulse rate.

## Claims

1. A ventilator system (1) comprising a ventilator (2), a nebulizer (3),
and an interface (4) comprising a controller (5), and the interface linking said ventilator with said nebulizer in a manner allowing control of at least some operations of the nebulizer by the ventilator controller,
wherein the interface (4) comprises a coupler (10) to the ventilator (2), a cable (12) linking said coupler to the interface controller (5), and a cable linking said interface controller (5) to a coupler (11) for fitting to the nebulizer (3),
wherein the interface controller (5) is configured to receive power from the ventilator and to use said power to drive the nebulizer,
wherein the interface controller (5) is configured to perform phasic delivery control of the nebulizer in response to commands from the ventilator,
wherein the interface controller (5) is configured to implement a time-out monitor in which it generates a command to cease nebulization if a start command has not been received from the ventilator controller before expiry of a time-out period,
wherein the interface controller (5) is configured to generate a nebulization re-start command each time a valid start command from the ventilator controller is received,
wherein the interface controller is configured to operate as a slave device, with a dosing regime determined by the ventilator controller.

2. A ventilator system as claimed in claim 1, wherein the interface controller (5) is configured to operate with a plurality of states which are transitioned according to commands from the ventilator.

3. A ventilator system as claimed in claim 2, wherein said commands include commands for a start-up state, an initialized state, and a nebulization state.

4. A ventilator system as claimed in claim 3, wherein the interface controller is configured to automatically transition from the nebulization state upon expiry of a timeout period.

5. A ventilator system as claimed in any of claims 1 to 4, wherein the interface controller (5) is configured to communicate with the ventilator according to any of a plurality of protocols.

6. A ventilator system as claimed in any of claims 1 to 5, in which the interface controller is configured to generate a command to suspend nebulization if a valid start command is not received within the timeout period, in which the interface controller reverts to an initialized state.

7. A ventilator system as claimed in any of claims 1 to 6, in which the nebulizer (3) comprises an aerosol generator (3b) with an aperture plate, which is vibrated at a drive frequency of 120 kHz to 150 kHz.

8. A ventilator system as claimed in claim 7, in which the controller is configured to chop an aerosol generator (3(b)) drive frequency at a pulse rate for control of the aerosol generator flowrate.

9. A ventilator system as claimed in any of claims 7 or 8, in which the system further comprises:
- a microcontroller comprising an integrated peripheral module configured to automatically change clock sources and power levels according to signals received from the ventilator controller, and configured to provide power and control signals via a cable (12), and
- a drive circuit configured to utilize a series inductor to generate an alternating AC voltage, the drive circuit comprising a high speed MOSFET driver controlled by a pulse width modulated signal from the microcontroller.

## Patentansprüche

1. Beatmungssystem (1), das ein Beatmungsgerät (2), einen Vernebler (3) und eine Schnittstelle (4) umfasst, die eine Steuerung (5) umfasst und das Beatmungsgerät auf eine Weise mit dem Vernebler verbindet, welche ein Steuern von zumindest einigen Operationen des Verneblers über die Beatmungsgerätsteuerung ermöglicht,
wobei die Schnittstelle (4) ein Kupplungsstück (10) für das Beatmungsgerät (2), ein das Kupplungsstück mit der Schnittstellensteuerung (5) verbindendes Kabel (12) und ein die Schnittstellensteuerung (5) mit einem Kupplungsstück (11) verbindendes Kabel zum Anstecken an den Vernebler (3) umfasst,
wobei die Schnittstellensteuerung (5) so konfiguriert ist, dass sie von dem Beatmungsgerät mit Strom versorgt wird und diesen zum Antreiben des Verneblers benutzt,
wobei die Schnittstellensteuerung (5) so konfiguriert ist, dass sie auf Befehle aus dem Beatmungsgerät hin eine phasische Abgabesteuerung des Verneblers durchführt,
wobei die Schnittstellensteuerung (5) so konfiguriert ist, dass sie eine Zeitlimitüberwachung umsetzt, bei der sie einen Befehl generiert, die Verneblung einzustellen, wenn vor Ablauf eines Zeitlimits kein Startbefehl aus der Beatmungsgerätsteuerung empfangen wurde,
wobei die Schnittstellensteuerung (5) so konfiguriert ist, dass sie jedesmal, wenn ein gültiger Startbefehl aus der Beatmungsgerätsteuerung empfangen wird, einen Verneblungsneustart-Befehl generiert,
wobei die Schnittstellensteuerung so konfiguriert ist, dass sie als Slave-Vorrichtung mit einem Dosiermodell arbeitet, das von der Beatmungsgerätsteuerung bestimmt wird.

2. Beatmungssystem nach Anspruch 1, wobei die Schnittstellensteuerung (5) so konfiguriert ist, dass sie mit mehreren Zuständen arbeitet, die Befehlen aus dem Beatmungsgerät entsprechend durchlaufen werden.

3. Beatmungssystem nach Anspruch 2, wobei zu den Befehlen Befehle für einen Einschaltzustand, einen initialisierten Zustand und einen Verneblungszustand gehören.

4. Beatmungssystem nach Anspruch 3, wobei die Schnittstellensteuerung so konfiguriert ist, dass sie bei Ablauf eines Zeitlimits automatisch aus dem Verneblungszustand wechselt.

5. Beatmungssystem nach einem der Ansprüche 1 bis 4, wobei die
Schnittstellensteuerung (5) so konfiguriert ist, dass sie entsprechend einem beliebigen von mehreren Protokollen mit dem Beatmungsgerät kommuniziert.

6. Beatmungssystem nach einem der Ansprüche 1 bis 5, bei dem die
Schnittstellensteuerung so konfiguriert ist, dass sie einen Befehl generiert, die Verneblung zu unterbrechen, wenn innerhalb des Zeitlimits, in dem die Schnittstellensteuerung in einen initialisierten Zustand zurückkehrt, kein gültiger Startbefehl empfangen wird.

7. Beatmungssystem nach einem der Ansprüche 1 bis 6, bei dem der Vernebler (3) einen Aerosolgenerator (3b) mit einer Lochplatte umfasst, die mit einer Antriebsfrequenz von 120 kHz bis 150 kHz vibriert.

8. Beatmungssystem nach Anspruch 7, bei dem die Steuerung so konfiguriert ist, dass sie zum Steuern der Aerosolgeneratordurchflussrate eine Antriebsfrequenz des Aerosolgenerators (3(b)) mit einer Pulsrate zerhackt.

9. Beatmungssystem nach einem der Ansprüche 7 oder 8, bei dem das System ferner Folgendes umfasst:
- einen Mikrocontroller mit einem integrierten Peripheriemodul, das so konfiguriert ist, dass es aus der Beatmungsgerätsteuerung empfangenen Signalen entsprechend automatisch Taktquellen und Leistungspegel ändert und über ein Kabel (12) Leistungs- und Steuersignale bereitstellt, und
- eine Antriebsschaltung, die so konfiguriert ist, dass sie eine in Reihe geschaltete Induktionsspule zum Erzeugen einer Wechselspannung benutzt, wobei die Antriebsschaltung einen schnellen MOSFET-Treiber umfasst, der über ein pulsbreitenmoduliertes Signal aus dem Mikrocontroller gesteuert wird.

## Revendications

1. Système de ventilation (1) comprenant un ventilateur (2), un nébuliseur (3), et une interface (4) comprenant un dispositif de commande (5), et l'interface reliant ledit ventilateur audit nébuliseur d'une manière permettant une commande d'au moins certaines opérations du nébuliseur par le dispositif de commande de ventilateur,
dans lequel l'interface (4) comprend un coupleur (10) au ventilateur (2), un câble (12) reliant ledit coupleur au dispositif de commande d'interface (5), et un câble reliant ledit dispositif de commande d'interface (5) à un coupleur (11) pour un ajustement au nébuliseur (3),
dans lequel le dispositif de commande d'interface (5) est configuré pour recevoir une puissance en provenance du ventilateur et pour utiliser ladite puissance pour entraîner le nébuliseur,
dans lequel le dispositif de commande d'interface (5) est configuré pour effectuer une commande de délivrance phasique du nébuliseur en réponse à des commandes en provenance du ventilateur,
dans lequel le dispositif de commande d'interface (5) est configuré pour mettre en œuvre un moniteur de temporisation dans lequel il génère une commande d'arrêt de nébulisation si une commande de démarrage n'a pas été reçue en provenance du dispositif de commande de ventilateur avant une expiration d'une période de temporisation,
dans lequel le dispositif de commande d'interface (5) est configuré pour générer une commande de redémarrage de nébulisation à chaque fois qu'une commande de démarrage valide est reçue en provenance du dispositif de commande de ventilateur,
dans lequel le dispositif de commande d'interface est configuré pour fonctionner en tant que dispositif esclave, avec un régime posologique déterminé par le dispositif de commande de ventilateur.

2. Système de ventilation selon la revendication 1, dans lequel le dispositif de commande d'interface (5) est configuré pour fonctionner avec une pluralité d'états entre lesquels la transition se fait selon des commandes en provenance du ventilateur.

3. Système de ventilation selon la revendication 2, dans lequel lesdites commandes comprennent des commandes pour un état de démarrage, un état initialisé, et un état de nébulisation.

4. Système de ventilation selon la revendication 3, dans lequel le dispositif de commande d'interface est configuré pour réaliser automatiquement une transition depuis l'état de nébulisation lors de l'expiration d'une période de temporisation.

5. Système de ventilation selon l'une quelconque des revendications 1 à 4, dans lequel le dispositif de commande d'interface (5) est configuré pour communiquer avec le ventilateur selon l'un quelconque d'une pluralité de protocoles.

6. Système de ventilation selon l'une quelconque des revendications 1 à 5, dans lequel le dispositif de commande d'interface est configuré pour générer une commande pour suspendre une nébulisation si une commande de démarrage valide n'est pas reçue durant la période de temporisation, dans lequel le dispositif de commande d'interface revient à un état initialisé.

7. Système de ventilation selon l'une quelconque des revendications 1 à 6, dans lequel le nébuliseur (3) comprend un générateur d'aérosol (3b) avec une plaque d'ouverture, qui est soumise à des vibrations à une fréquence d'entraînement de 120 kHz à 150 kHz.

8. Système de ventilation selon la revendication 7, dans lequel le dispositif de commande est configuré pour hacher une fréquence d'entraînement de générateur d'aérosol (3(b)) à une cadence d'impulsion pour commander le débit d'écoulement de générateur d'aérosol.

9. Système de ventilation selon l'une quelconque des revendications 7 ou 8, dans lequel le système comprend en outre :
- un micro-contrôleur comprenant un module périphérique intégré configuré pour changer automatiquement des sources d'horloge et des niveaux de puissance selon des signaux reçus en provenance du dispositif de commande de ventilateur, et configuré pour fournir des signaux de puissance et de commande par l'intermédiaire d'un câble (12), et
- un circuit d'entraînement configuré pour utiliser un inducteur série pour générer une tension CA alternative, le circuit d'entraînement comprenant un élément d'entraînement MOSFET haute vitesse commandé par un signal modulé en largeur d'impulsion provenant du micro-contrôleur.
